Europäisches Patentamt

(19) European Patent Office

Office européen des brevets

(11) Veröffentlichungsnummer : **0 156 024**
**B1**

(12) **EUROPÄISCHE PATENTSCHRIFT**

(45) Veröffentlichungstag der Patentschrift :
23.03.88

(21) Anmeldenummer : 84115756.3

(22) Anmeldetag : 18.12.84

(51) Int. Cl.⁴ : **A 61 B 6/00**, H 01 L 31/00,
G 01 T 1/24

(54) **Detektorsystem.**

(30) Priorität : 09.03.84 DE 3408681

(43) Veröffentlichungstag der Anmeldung :
02.10.85 Patentblatt 85/40

(45) Bekanntmachung des Hinweises auf die Patenterteilung : 23.03.88 Patentblatt 88/12

(84) Benannte Vertragsstaaten :
DE FR

(56) Entgegenhaltungen :
DE-A- 2 546 451
DE-A- 2 806 858
US-A- 3 674 712
US-A- 3 974 388
US-A- 4 174 481
IEEE Transactions on Nuclear Science, vol. NS-27, no. 1, Februar 1980, San Francisco, USA, Y. NARUSE et al. "Multichannel semiconductor detectors for X-Ray transmission computed tomography", S. 252-257
IEEE Transactions on Nuclear Science, vol. NS-20, no. 1, Februar 1973, Miami, USA, R.H. Pehl et al., "Operational characteristics of germanium detectors at highes temperatures", S. 494-499

(73) Patentinhaber : Siemens Aktiengesellschaft Berlin und München
Wittelsbacherplatz 2
D-8000 München 2 (DE)

(72) Erfinder : Glasow, Peter. Dipl.-Phys.
Fichtestrasse 14
D-8520 Erlangen (DE)
Erfinder : Rühle, Wolfgang, Dr.
Georg-Krauss-Strasse 2
D-8520 Erlangen (DE)

**Beschreibung**

Die Erfindung bezieht sich auf ein Detektorsystem für ionisierende Strahlen, insbesondere Röntgenstrahlen, für die Computer-Tomographie CT und -Radiographie CR mit einer Vielzahl von flachen Detektoren, die mit ihren Flachseiten nebeneinander angeordnet sind. Wenigstens ein Teil einer ihrer Schmalseiten ist zum Empfang der Strahlen vorgesehen. Ihre Flachseiten sind jeweils mit einer Elektrode versehen, von denen einer ein sperrender Übergang zugeordnet ist.

Es sind Anordnungen zum Herstellen eines Körperschnittbildes bekannt, bei denen ein fächerförmiges Bündel ionisierender Strahlen die Bildelemente in der Körperschnittebene nacheinander in verschiedenen Richtungen durchsetzt. Dem Strahlenbündel ist eine Detektor-anordnung mit einer Vielzahl von in der Körperschnittebene nebeneinander angeordneten Detektoren zugeordnet. Die Umsetzung der verschiedenen Einzelaufnahmen der Körperelemente und deren Zuordnung zu dem entsprechenden Bildelement des herzustellenden Körperschnittbildes erhält man mit Hilfe einer Elektronik, die einen Computer enthält. Diese bekannten Anordnungen, bei denen die Strahlungsquelle mit dem Detektorsystem in der Körperschnittebene schrittweise um den Körper gedreht wird, sind bekannt unter der Bezeichnung Computer-Tomographen (US-Patent 3 974 388).

Es sind ferner Anordnungen zum Herstellen von Röntgen-Schattenbildern eines Aufnahmeobjektes, vorzugsweise eines menschlichen Körpers, bekannt, bei der ebenfalls einem fächerförmigen Bündel ionisierender Strahlen ein Detektorsystem zugeordnet ist, bei dem jedoch der abzubildende Körper relativ zum Strahlenbündel nach jeder Durchstrahlung schrittweise senkrecht zum Fächer des Strahlenbündels bewegt wird. Den in der Strahlungsebene angeordneten Detektoren werden somit jeweils nacheinander die Absorptionswerte einander paralleler Schichten des Körpers zugeführt. Solche Anordnungen zum Herstellen von Röntgen-Schattenbildern mit einer Bewegung senkrecht zum Fächer des Strahlenbündels sind bekannt unter der Bezeichnung Computer-Radiographen (US-Patent 4 174 481).

Bei der Computer-Tomographie CT und der Computer-Radiographie CR erfaßt ein Detektorsystem mit mehreren 100, insbesondere mehr als 1000, Detektoren die durch den menschlichen Körper geschwächte ionisierende Strahlung, insbesondere Röntgen- oder $\gamma$-Strahlung. Die entsprechende Detektoreinrichtung muß nun diese durch die einzelnen Körperteile untershiedlich geschwächte Strahlung möglichst vollständig nachweisen, also absorbieren. Ferner soll das elektronische Rauschen des Detektors mit der entsprechenden Verstärkerelektronik klein sein gegenüber dem Quantenrauschen $\sqrt{N}$ der in der Meßzeit auftretenden minimalen Röntgenquantenzahl N, d. h. bei maximaler Schwächung. Die verwendeten Detektoren sollen außerdem insbesondere für ein schnelles Computer-Tomographie-Gerät mit kontinuierlicher Röntgenstrahlung ein schnelles Zeitverhalten zeigen und es soll darüber hinaus kein Nachleuchten oder Nachstrom auftreten, da diese zur Verfälschung des Röntgenbildes führen können.

Es ist bekannt, daß für die Computer-Tomographie und Computer-Radiographie Detektorsysteme mit einer Szintillator-Photodiodenkombination mit Silizium-Photodioden verwendet werden können. Ferner sind Xenon-Kammern als Detektorsystem für Röntgenstrahlen geeignet. Für ein hochauflösendes Computer-Tomographie-Gerät mit kontinuierlicher Röntgenstrahlung erfüllt keiner der Detektoren die erwähnten Bedingungen. Das in einer Szintillator-Photodiodenkombination im allgemeinen verwendete Cäsiumjodid CsJ zeigt zwar hohe Signale, jedoch störendes Nachleuchten. Weitere bekannte Szintillatoren haben um einen Faktor 4 bis 5 kleinere Signale. Xenon ermöglicht nur eine unvollständige Absorption der Röntgenstrahlen. Weitere Probleme bei Xenon sind hohe Drücke, hohe Felder bei kleinem Elektrodenabstand und Probleme beim Fokuswackeln der Röntgenröhre.

Der Erfindung liegt nun die Aufgabe zugrunde, ein Detektorsystem für die Computer-Tomographie und -Radiographie anzugeben, das alle die erwähnten Bedingungen erfüllt.

Ein Detektorsystem für die Computer-Tomographie kann Halbleiterdetektoren mit einem flachen Halbleiterkörper aus n-leitendem Silizium enthalten, deren Flachseiten jeweils mit einer Elektrode versehen sind, von denen eine einen sperrenden Übergang bildet, der als Metall-Halbleiterübergang ausgeführt ist (DE-A-2 806 858). Eine der Stirnseiten des Halbleiterkörpers mit einer Breite von etwa 1,5 mm und einer Länge von etwa 17 mm ist zum Empfang der Strahlung vorgesehen. Die Tiefe der Halbleiterkörper in der Strahlungsrichtung ist verhältnismäßig groß gewählt und kann etwa 20 mm betragen. (IEEE Trans. on Nucl. Science, Vol. 27, Nr. 1, Febr. 1980, S. 252-257). Silizium absorbiert jedoch nur einen verhältnismäßig geringen Teil ionisierender Strahlen. Auch Eine Tiefe von beispielsweise etwa 40 mm in Richtung der ankommenden Strahlen reicht zur vollständigen Absorption noch nicht aus.

Es ist ferner bekannt, daß Detektorsysteme mit Halbleiterdetektoren aus sogenanntem Reinstgermanium oder hochreinem Germanium mit einer sehr geringen Dotierungskonzentration von beispielsweise nur etwa $10^{11}$ bis $10^9$ Atomen/cm$^3$ für die Computer-Tomographie geeignet sind. In einer bekannten Ausführungsform eines derartigen Detektorsystems ist jedoch wegen der schmalen Bandlücke der Germanium-Detektoren ein Betrieb des Systems bei kryogener Temperatur um 80 K und im Vakuum vorgesehen.

Es ist zwar bekannt, daß Detektoren für nukleare Strahlung aus Reinstgermanium mit einem großen Volumen in einer besonderen Ausführungsform bei höherer Temperatur gelagert werden können (DE-OS 25 46 451). Es ist jedoch durch präzise Messungen belegt und durch Diagramme veranschaulicht worden, daß Germaniumdetektoren nur bei Temperaturen unterhalb 150 K betrieben werden können (IEEE Trans. on Nucl. Science T-NS 73, Febr., S. 494-499 (9 D 02)).

Die Erfindung beruht nun auf der Erkenntnis, daß Reinstgermanium unter besonders gewählten Bedingungen auch bei Raumtemperatur, vorzugsweise jedoch bei mäßiger Kühlung, auf beispielsweise 240 K, d. h. ohne flüssigen Stickstoff, auch den höchsten Anforderungen eines hochauflösenden Computer-Tomographie-Gerätes mit kontinuierlicher Röntgenstrahlung genügt. Die Erfindung besteht somit darin, daß für ein Detektorsystem der eingangs genannten Art Detektoren mit einem Grundmaterial aus hochreinem Germanium vorgesehen sind, deren Volumen V so gewählt ist, daß bei einer physikalisch gegebenen Eigenleitungskonzentration $n_i$ des Germaniums und einer technologisch vorgegebenen Trägerlebensdauer $\tau_i$ des Germaniums sowie einer Dotierungskonzentration von höchstens $10^{13}$ Atomen/cm$^3$ der Beitrag des Generations-Rekombinationsstromes $I_G$ zum Schrotrauschen $\sqrt{\overline{i^2}}$ der Detektoren 12 auch bei höheren Temperaturen als bei Detektoren 12 für ionisierende Strahlen 2 üblich wesentlich kleiner ist als das Quantenrauschen der Strahlen 2. Mit diesem Detektorsystem erhält man eine gute Ortsauflösung sowie ein gutes Signal-Rauschverhältnis und man benötigt kein Vakuum und für einen spannungslosen Betrieb des Detektorsystems auch keine Kühlung. In einer besonders vorteilhaften Gestaltungsform des Detektorsystems sind die Detektoren mit einem durch Ionenimplantation hergestellten sperrenden Übergang versehen. Damit bleibt nicht nur der Generations-Rekombinationsstrom $I_G$, sondern auch der Diffusionsstrom $I_D$ klein, so daß auch sein Beitrag zum Schrotrauschen bei höheren Temperaturen klein bleibt. Ferner können in einer bevorzugten Ausführungsform des Detektorsystems die Schmalseiten der Detektoren einer Nachbehandlung unterzogen werden, welche die Oberflächenströme begrenzt. Weitere besonders vorteilhafte Gestaltungsformen der Detektoranordnung sowie neue Betriebsverfahren sind Gegenstand der Unteransprüche.

Die Erfindung beruht auf folgenden Überlegungen. Das Quantenrauschen QR der Röntgenquanten hat einen wesentlichen Einfluß auf die Bildqualität. Die mittlere Leistung einer kontinuierlich strahlenden Röntgenröhre wird im allgemeinen 10 kW nicht wesentlich überschreiten. Bei einer mittleren Energie von $h\gamma = 80$ keV werden somit im Mittel $N = 1,25 \cdot 10^5$ Quanten/mm$^2$ und ms in 1 m Entfernung ankommen. Die stochastisch unabhängigen Quanten unterliegen der Poissonstatistik, so daß die Streuung $\sqrt{N}$ beträgt. Eine Schwächung des Röntgenstrahls bei der Computer-Radiographie im Patienten um etwa den Faktor 100 sowie eine Integrationszeit von 10 ms sind üblich. Nun sollen in einem guten Detektor wenigstens 80 % der in den Detektor eintretenden Quanten absorbiert werden. Demnach ist $N = 10^4$ Quanten/mm$^2$ Detektorfläche und Integrationszeit. Damit ist das Quantenrauschen $QR = \sqrt{N} = 10^2$ und das relative Quantenrauschen beträgt somit 1 %. Mit einem schlechten Detektor, der nur etwa 20 % der Quanten absorbiert, erhält man $N = 2,5 \cdot 10^3$ und $QR = 50$ ; das relative Quantenrauschen QR beträgt somit 2 % und man erhält ein Bild mit entsprechend geringer Qualität.

Für schlechte Detektoren könnte nun eine mehrfache Integrationszeit gewählt werden. Damit würde jedoch die Dosis für den Patienten entsprechend höher und die Aufnahmezeit verlängert ; es würden sich deshalb Bewegungsunschärfen entsprechend bemerkbar machen. Für den Detektor muß somit ein Material gewählt werden, mit dem man bei entsprechender Absorptionstiefe eine möglichst vollständige Absorption der Röntgenquanten erhält.

Die Absorption von Röntgenstrahlen hängt bekanntlich von der Kernladungszahl und der Dichte des verwendeten Materials ab. Sie folgt nicht einem einfachen Exponentialgesetz, da es sich um ein kontinuierliches Strahlenspektrum, vorzugsweise einem Röntgenspektrum, handelt, das im Detektor aufgehärtet wird. Die Absorption ist auch abhängig vom Spektrum vor dem Detektor, also von der Röhrenspannung, von Kupferfiltern und davon, ob das Spektrum durch den Patienten aufgehärtet ist. Die Schwächung des Röntgenstrahls erfolgt nicht nur durch Photo-, sondern insbesondere bei leichten Elementen auch durch Compton-Effekt. Es wurde nun gefunden, daß bei Detektoren aus Reinstgermanium Ge nur eine Tiefe in Richtung der ankommenden Strahlung von nicht wesentlich mehr als 8 mm zur vollständigen Absorption ausreicht.

Zur weiteren Erläuterung der Erfindung wird auf die Zeichnung Bezug genommen, in der eine Anordnung zum Abbilden von Teilen eines Körpers mit einem Detektorsystem nach der Erfindung schematisch veranschaulicht ist.

In der dargestellten Ausführungsform für die Computer-Tomographie CT ist einem fächerförmigen Bündel ionisierender Strahlen 2, vorzugsweise γ-Strahlen, insbesondere Röntgenstrahlen, die ein Aufnahmeobjekt, vorzugsweise einen menschlichen Körper 6, das auf einer Platte 8 liegt, durchsetzen, ein Detektorsystem 10 zugeordnet, das Detektoren 12 mit einem Grundmaterial aus Reinstgermanium enthält. Das Detektorsystem 10 kann vorzugsweise mehrere 100, insbesondere mehr als 1000, Detektoren 12 enthalten, die mit ihren Flachseiten nebeneinander angeordnet sind und von denen zur Vereinfachung lediglich ein einzelner Detektor 12 dargestellt ist. Ein Teil der oberen Schmalseite der flachen Detektoren 12, deren Flachseiten rechteckförmig oder auch quadratisch gestaltet sein können, ist zum Empfang der Strahlen 2 vorgesehen. Der Detektor enthält einen Halbleiterkörper mit einem Grundmaterial aus hochreinem p- oder n-Germanium, dessen Konzentration an elektrisch aktiven Verunreinigungen kleiner als $10^{13}$ Atome/cm$^3$, vorzugsweise kleiner als $10^{12}$ Atome/cm$^3$, ist und insbe-

sondere nur $10^9$ bis $10^{11}$ Atome/cm$^3$ beträgt. Die Trägerlebensdauer $\tau_i$ im Grundmaterial ist groß und beträgt beispielsweise wenigstens 0,1 ms, vorzugsweise wenigstens 1 ms, insbesondere mindestens 10 ms. Der Halbleiterkörper ist an seinen beiden Flachseiten mit Elektroden versehen, die jeweils über einen elektrischen Anschlußleiter 16 bzw. 17 mit einer in der Figur nicht dargestellten Elektronik verbunden sind. Von den beiden Elektroden bildet eine einen sperrenden Übergang, der vorzugsweise ein pn-Übergang sein kann und beispielsweise durch Diffusion, insbesondere durch Ionenimplantation, hergestellt sein kann. Der sperrende Übergang wird so hergestellt, daß sowohl der Diffusionsstrom $I_D$ als auch der Generations-Rekombinationsstrom $I_G$ möglichst klein bleiben, damit das Schrotrauschen $\sqrt{i^2}$ des Sperrstromes $I_S$ ebenfalls minimal bleibt.

Als Gegendotierungsstoff zur Herstellung des pn-Überganges kann für p-leitendes Germanium beispielsweise Arsen As, Antimon Sb oder Lithium Li, vorzugsweise Phosphor P, gewählt werden. Für n-leitendes Germanium ist beispielsweise Aluminium Al, Gallium Ga, Indium In oder Beryllium Be, vorzugsweise Bor B, geeignet. Für den sperrfreien Rückkontakt auf der gegenüberliegenden Flachseite, einen sogenannten BSF-Kontakt (back surface field), mit einer hochdotierten Schicht mit abstoßender Wirkung für Minoritätsladungsträger kann in p-leitendes Germanium beispielsweise Aluminium Al, Gallium Ga oder Indium In, oder Beryllium Be, vorzugsweise Bor B, und in n-leitendes Germanium beispielsweise Arsen As, Antimon Sb oder Lithium Li, vorzugsweise Phosphor P, implantiert werden. Als sperrender Übergang ist auch ein Metall-Halbleiterübergang geeignet.

Zur Befestigung der Anschlußleiter 16 bzw. 17 können die Elektroden jeweils mit einem Metallkontakt versehen sein, von denen nur einer in der Figur angedeutet und mit 18 bezeichnet ist und die beispielsweise aus Palladium, Aluminium, Gold oder Nickel-Chrom bestehen und beispielsweise aufgedampft oder auch aufgeklebt sein können.

Das Volumen des Halbleiterkörpers wird so klein gewählt, daß das Schrotrauschen durch den Generations-Rekombinationsstrom auch bei höheren Temperaturen der Detektoren 12 wesentlich kleiner ist als das Quantenrauschen der Strahlen 2. Die Breite b wird wesentlich bestimmt durch die Anzahl der Detektoren 12 und die Breite des Fächers der Strahlen 2 und sie kann vorzugsweise etwa 0,4 bis 1,5 mm, insbesondere etwa 0,5 mm, betragen. Die Länge a der Detektoren 12 wird bestimmt durch einen in der Figur nicht dargestellten Kollimator der Strahlungsquelle 4 und sie kann in verhältnismäßig weiten Grenzen von beispielsweise etwa 12 bis 25 mm, insbesondere etwa 15 mm für die Computer-Tomographie und etwa 1 bis 4 mm, insbesondere etwa 2 bis 3 mm, für die Computer-Radiographie gewählt werden. Dem Kollimator der Strahlungsquelle 4 ist eine Einrichtung zugeordnet, welche die wirksame

Länge $a_1$ der Detektoren 12 ausblendet und die in der Figur zur Vereinfachung als Blende 19 mit zwei Blechen angedeutet ist. Die Öffnung der Blende 19 und damit die wirksame Länge $a_1$ der Detektoren 12 kann vorzugsweise veränderbar sein, beispielsweise zwischen etwa 2 bis 12 mm, vorzugsweise etwa 2 bis 8 mm für die Computer-Tomographie und etwa 1 bis 8 mm für die Computer-Radiographie. Die Tiefe t der Detektoren 12 wird so gewählt, daß man eine nahezu vollständige Absorption der Strahlen 2 im Volumen des Detektors 12 erhält und sie wird für die Computer-Tomographie CT im allgemeinen wesentlich weniger als 15 mm, vorzugsweise höchstens 12 mm, insbesondere höchstens 10 mm, gewählt. Mit einer Tiefe t des Grundmaterials der Detektoren 12 von nur 8 mm werden bereits etwa 90 % der Strahlen 2 absorbiert. Für die Computer-Radiographie CR wird eine Tiefe t von etwa 3 bis 8 mm, vorzugsweise etwa 4 bis 5 mm, gewählt.

Die Schmalseiten der Detektoren 12 können vor dem Einbau in das Detektorsystem 10 vorzugsweise einer Behandlung unterzogen werden, welche die Oberflächenströme $I_O$ und damit ihren Beitrag zum Schrotrauschen $\sqrt{i_2}$ reduziert. Zu diesem Zweck können die Schmalseiten insbesondere einer Nachätzung, beispielsweise mit einer Mischung aus Fluß- und Salpetersäure, unterzogen werden. In einer besonderen Ausführungsform der Detektoren werden diese nachgeätzten Schmalseiten mit einem Überzug 20 aus passivierendem Material, beispielsweise aus Siliziumnitrit $Si_3N_4$ oder Siliziumoxid $SiO_2$ oder aus amorphem Germanium, vorzugsweise einer Klebeschicht, insbesondere aus Cyanacrylat, versehen.

Das Signal wächst zwar mit der Zahl der Quanten, also auch mit der Größe des absorbierenden Volumens, und ein größerer Detektor hat somit auch eine größere Ansprechempfindlichkeit, bezogen auf die Strahlungsquelle. Proportional dem Volumen wächst jedoch auch das Schrotrauschen des Generations- und Rekombinationsstromes $I_G$. Mit dem angegebenen geringen Volumen bleibt das Schrotrauschen wesentlich kleiner als das Quantenrauschen und man erhält eine Absorption von etwa 90 % der Strahlen.

Ein Sperrstrom $I_s$ in Dioden ergibt ein Schrotrauschen

$$\sqrt{i^2} = \sqrt{2\,e \cdot I_s \cdot \Delta f}\,,$$

wobei e die Elementarladung der Quanten und $\Delta f$ die Bandbreite ist. Im spannungslosen Zustand fließt zusätzlich zum Sperrstrom ein gleich großer Feldstrom. Die Schwankungsquadrate addieren sich, so daß man für spannungslosen Betrieb ein Schrotrauschen

$$\sqrt{i^2} = \sqrt{4\,e \cdot I_s \cdot \Delta f}$$

erhält. Wegen seiner kleinen Energielücke ergibt Germanium bei Raumtemperatur bereits hohe Sperrströme. Diese setzen sich zusammen aus dem Generations-Rekombinationsstrom $I_G$ im ganzen Volumen, dem Diffusionsstrom $I_D$ der Minoritätsladungsträger im p- und/oder n-Kontaktgebiet und dem Oberflächenstrom $I_O$, und man erhält

$$I_S = I_G + I_D + I_O,$$

wobei

$$I_G = \frac{e \cdot n_i \cdot V}{2\,\tau_i}$$

und beispielsweise für ein n-Kontaktgebiet

$$I_D = e \sqrt{\frac{D_p}{\tau_p}} \cdot \frac{n_i^2}{N_D} \cdot F$$

und entsprechend für ein p-Kontaktgebiet

$$I_D = e \sqrt{\frac{D_n}{\tau_n}} \cdot \frac{n_i^2}{N_A} \cdot F$$

ist.

Darin ist V das Volumen, F die Fläche der Diode, $D_p$ die Diffusionskonstante der Löcher im n-Kontaktgebiet, $D_n$ die Diffusionskonstante der Elektronen im p-Kontaktgebiet, $\tau_p$ die Lebensdauer der Löcher im n-Kontaktgebiet und $\tau_n$ die Lebensdauer der Elektronen im p-Kontaktgebiet. $N_D$ ist die Donatorenkonzentration im n-Kontaktgebiet und $N_A$ die Akzeptorenkonzentration im p-Kontaktgebiet und $n_i$ die Eigenleitungskonzentration des Germaniums und $\tau_i$ die Trägerlebensdauer in den i-Gebieten. Der Diffusionsstrom $I_D$ und der Oberflächenstrom $I_O$ können durch besondere Präparation begrenzt werden ; dagegen hat der Generations-Rekombinationsstrom $I_G$ einen festen minimalen Wert, der durch die Trägerlebensdauer $\tau_i$ im Grundmaterial bestimmt wird. Die maximale Betriebstemperatur der Detektoren 12 ergibt sich somit aus einem Vergleich des Generations-Rekombinationsstroms $I_G$ mit dem Quantenrauschen QR.

In der dargestellten Anordnung für die Computer-Tomographie CT mit auf einem Kreisbogen der fächerförmigen Strahlung nebeneinander angeordneten Detektoren 12 beträgt die an den Detektoren 12 ankommende minimale Quantenzahl beispielsweise $N_{min} = 1{,}55 \cdot 10^3$ Quanten/2 ms Integrationszeit und 2 mm wirksamer Länge $a_1$ der Stirnfläche, die vorzugsweise mit Hilfe der Blende 19 aus der Länge a ausgeblendet sein kann. Die mittlere Energie E des durch den Körper 6 aufgehärteten Spektrums der Strahlen 2 beträgt beispielsweise 104 keV und der Detektor 12 absorbiert bei einer Tiefe t von beispielsweise 8 mm etwa 90 % der Strahlen 2. Mit einer Eigenleitungskonzentration des Germaniums $n_i = 2{,}4 \cdot 10^{13}\,\text{cm}^{-3}$ bei Raumtemperatur und einer Trägerlebensdauer $\tau_i = 10^{-2}$ s sowie einer Brandbreite $\Delta f = 100$ Hz und einer Paarbildungsenergie $E_i = 3$ eV für Detektoren 12 mit einem

Volumen $0{,}7 \cdot 13{,}5 \cdot 8$ mm$^3$ erhält man einen Generations-Rekombinationsstrom $I_G = 1{,}5 \cdot 10^{-5}$ A und einen minimalen Signalstrom $i_{min}$ von $3{,}9 \cdot 10^{-9}$ A, die ein Schrotrauschen $\sqrt{i_2^2} = 3{,}1 \cdot 10^{-11}$ A und ein Quantenrauschen von $1{,}1 \cdot 10^{-10}$ A ergeben. Das Schrotrauschen beträgt somit bei Raumtemperatur nur etwa 1/3 des Quantenrauschens. Zur Verminderung des Sperrstromes, insbesondere des $n_i^2$-proportionalen Stromanteils kann es zweckmäßig sein, die Detektoranordnung 10 mäßig zu kühlen, vorzugsweise auf eine Temperatur von etwa 240 K und unter Umständen auch auf eine Temperatur von etwa 200 K.

Der Aufbau des Detektorsystems 10 mit getrennten Detektoren hat ferner den Vorteil, daß ein spannungsloser Betrieb auch bei höheren Temperaturen möglich ist. Wenn die einzelnen Detektoren mit ihrem zugeordneten Kollimator jeweils eine getrennte Baueinheit bilden, können die einzelnen Detektoren ohne großen Aufwand ausgetauscht werden. Durch die Abstände werden außerdem ungünstige Effekte durch ein gegebenenfalls auftretendes Fokuswackeln der Röntgenröhre der Strahlungsquelle 4 und ungünstige Effekte durch gebogene Kollimatorbleche vermieden.

In der praktischen Ausführungsform eines Detektorsystems für die Computer-Tomographie CT können die Detektoren 12 vorzugsweise in der Ebene des fächerförmigen Strahlenbündels in wenigstens annähernd gleichem Abstand zur Strahlungsquelle 4 auf einem Kreisbogen des Fächers der Strahlen 2 derart nebeneinander angeordnet sein, daß die Flachseiten jeweils eines der Detektoren 12 und des zugeordneten Kollimators parallel zu den Strahlen angeordnet sind, die an der Stirnseite dieses Detektors einfallen.

Ein Detektorsystem für die Computer-Radiographie CR ist dagegen im allgemeinen nicht gekrümmt und die Detektoren sind auf einer Sehne des Fächers der Strahlen und mit ihren Flachseiten sowie ihrem zugeordneten Kollimator parallel zu den einfallenden Strahlen angeordnet.

**Patentansprüche**

1. Detektorsystem (10) für ionisierende Strahlen für die Computer-Tomographie CT und Computer-Radiographie CR mit einer Vielzahl von flachen Detektoren (12), die mit ihren Flachseiten nebeneinander angeordnet sind und deren eine Schmalseite wenigstens teilweise zum Empfang der Strahlen vorgesehen ist und deren Flachseiten jeweils mit einer Elektrode versehen sind, von denen einer ein sperrender Übergang zugeordnet ist, dadurch gekennzeichnet, daß Detektoren (12) aus hochreinem Germanium vorgesehen sind, deren Volumen (V) so gewählt ist, daß bei einer physikalisch gegebenen Eigenleitungskonzentration ($n_i$) des Germaniums und einer technologisch vorgegebenen Trägerlebensdauer ($\tau_i$) des Germaniums sowie einer Dotierungskonzentration von höchstens $10^{13}$ Atomen/cm$^3$ der Beitrag des

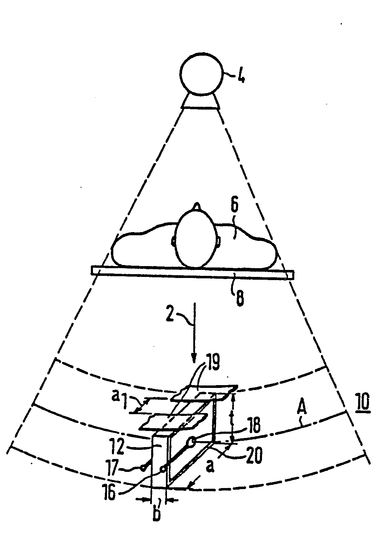

Generations-Rekombinationsstromes ($I_G$) zum Schrotrauschen ($\sqrt{i_2}$) der Detektoren (12) auch bei höheren Temperaturen als bei Detektoren (12) für ionisierende Strahlen (2) üblich wesentlich kleiner ist als das Quantenrauschen der Strahlen (2) (Figur 1).

2. Detektorsystem nach Anspruch 1, dadurch gekennzeichnet, daß der sperrende Übergang durch Ionenimplantation hergestellt ist.

3. Detektorsystem nach Anspruch 1 oder 2, dadurch gekennzeichnet, daß die Tiefe (t) der Detektoren (12) in Richtung der Strahlen (2) höchstens 15 mm, vorzugsweise höchstens 12 mm, insbesondere höchstens 10 mm, beträgt.

4. Detektorsystem nach einem der Ansprüche 1 bis 3, dadurch gekennzeichnet, daß die Schmalseiten der Detektoren (12) vor deren Einbau in das Detektorsystem (10) einer Nachätzung unterzogen sind.

5. Detektorsystem nach Anspruch 4, dadurch gekennzeichnet, daß die geätzten Schmalseiten der Detektoren (12) mit einem Überzug (20) aus einem passivierenden Material versehen sind.

6. Detektorsystem nach Anspruch 5, dadurch gekennzeichnet, daß der passivierende Überzug (20) aus einem Cyanacrylat besteht.

7. Detektorsystem nach einem der Ansprüche 1 bis 6, dadurch gekennzeichnet, daß die Konzentration an elektrisch aktiven Verunreinigungen im Grundmaterial der Detektoren (12) höchstens $10^{12}$ Atome/cm$^3$ beträgt.

8. Detektorsystem nach Anspruch 7, dadurch gekennzeichnet, daß die Konzentration an elektrisch aktiven Verunreinigungen im Grundmaterial annähernd $10^{10}$ Atome/cm$^3$ beträgt.

9. Verfahren zum Betrieb eines Detektorsystems nach einem der Ansprüche 1 bis 8, dadurch gekennzeichnet, daß das Detektorsystem bei einer Temperatur oberhalb 200 K betrieben wird.

10. Verfahren zum Betrieb eines Detektorsystems nach Anspruch 11, dadurch gekennzeichnet, daß das Detektorsystem bei einer Temperatur oberhalb 240 K betrieben wird.

## Claims

1. A detector system (10) for ionising radiation for computer tomography CT and computer radiography CR having a plurality of flat detectors (12) arranged with their flat faces adjacent to one another, at least one edge thereof being arranged, at least in part, to receive radiation and the flat faces each being provided with an electrode, with one of which a reverse-bias junction is associated, characterised in that detectors (12) of high-purity germanium are provided, the volume (V) of which is selected so that at a physically determined intrinsic conduction concentration ($n_i$) of the germanium and a technologically predetermined carrier life ($\tau_i$) of the germanium and a doping concentration of at most $10^{13}$ atoms/cm$^3$ the contribution of the generation-recombination current ($I_G$) to the shot noise ($\sqrt{i^2}$) of the detectors (12) is substantially less, even at higher temperatures than are usual for detectors (12) for ionising radiation, than the quantum noise of the radiation (2) (Fig. 1).

2. A detector system according to claim 1, characterised in that the reverse-bias junction is produced by ion implantation.

3. A detector system according to claim 1 or claim 2, characterised in that the depth (t) of the detector (12) in the direction of the radiation (2) is at most 15 mm, preferably at most 12 mm, and especially at most 10 mm.

4. A detector system according to any one of claims 1 to 3, characterised in that the edges of the detectors (12) are subjected to a post-etching step before they are assembled into the detector system.

5. A detector system according to claim 4, characterised in that the etched edges of the detectors (12) are provided with a coating (20) of a passivating material.

6. A detector system according to claim 5, characterised in that the passivating coating (20) consists of a cyanoacrylate.

7. A detector system according to any one of claims 1 to 6, characterised in that the concentration of electrically active impurities in the basis material of the detectors (12) is at most $10^{12}$ atoms/cm$^3$.

8. A detector system according to claim 7, characterised in that the concentration of electrically active impurities in the basis material is about $10^{10}$ atoms/cm$^3$.

9. A process for operating a detector system according to any one of claims 1 to 8, characterised in that the detector system is operated at a temperature above 200 K.

10. A process for operating a detector system according to claim 11, characterised in that the detector system is operated at a temperature above 240 K.

## Revendications

1. Système de détection (10) pour des rayonnements ionisants pour la tomodensitométrie CT et la radiographie CR assistées par ordinateur, comportant une multiplicité de détecteurs plats (12), dont les côtés plats sont disposés côte-à-côte et dont un petit côté est prévu au moins en partie pour la réception du rayonnement tandis que ses côtés plats sont munis d'électrodes respectives, à l'une desquelles est associée une jonction réalisant un blocage, caractérisé par le fait qu'il est prévu des détecteurs (12) constitués par du germanium très pur et dont le volume (V) est choisi de telle sorte que, pour un degré de conduction intrinsèque ($n_i$), donné physiquement, pour une durée de vie ($\tau_i$) des porteurs, prédéterminée par la technologie, dans le germanium ainsi que pour une concentration de dopage égale au maximum à $10^{13}$ atomes/cm$^3$, la participation du courant de génération-recombinaison ($I_G$) au bruit de grenaille ($\sqrt{i^2}$) des détecteurs (12), même pour des températures supérieures aux températures usuelles intervenant dans des détecteurs (12) pour des rayonnements ionisants (2), est nettement plus faible que celle du bruit quantique du rayonnement (2) (figure 1).

2. Système de détection suivant la revendication 1, caractérisé par le fait que la jonction établissant un blocage est formée par implantation ionique.

3. Système de détection suivant la revendication 1 ou 2, caractérisé par le fait que l'épaisseur (t) du détecteur (12) dans la direction du rayonnement (2) est égale au maximum à 4 mm et de préférence est égale au maximum à 12 mm et notamment est égale au maximum à 10 mm.

4. Système de détection suivant l'une des revendications 1 à 3, caractérisé par le fait que les petits côtés du détecteur (12) sont soumis, avant leur insertion dans le système de détection (10), à une post-corrosion.

5. Système de détection suivant la revendication 4, caractérisé par le fait que les petits côtés corrodés du détecteur (12) sont munis d'un revêtement (20) réalisé en un matériau produisant une passivation.

6. Système de détection suivant la revendication 5, caractérisé par le fait que le revêtement (20) réalisant une passivation est constitué par une cyanacrylate.

7. Système de détection suivant l'une des revendications 1 à 6, caractérisé par le fait que la concentration des impuretés actives du point de vue électrique dans le matériau de base des détecteurs (12) est égale au maximum à $10^{12}$ atomes/cm$^3$.

8. Système de détection suivant la revendication 7, caractérisé par le fait que la concentration en impuretés active du point de vue électrique dans le matériau de base est égale approximativement à $10^{10}$ atomes/cm$^3$.

9. Procédé de mise en œuvre d'un système de détection suivant l'une des revendications 1 à 8, caractérisé par le fait qu'on fait fonctionner le système de détection à une température supérieure à 200 °K.

10. Procédé de mise en œuvre d'un système de détection suivant la revendication 11, caractérisé par le fait qu'on fait fonctionner le système de détection à une température supérieure à 240 °K.